# EUROPEAN PATENT APPLICATION

(11) **EP 4 566 547 A1**
(43) Date of publication of application: **11.06.2025**
(21) Application number: 24215546.3
(22) Date of filing: 26.11.2024
(51) Int. Cl.: A61B 8/08, A61B 17/34

(54) **PROCESSOR DEVICE AND OPERATION METHOD OF PROCESSOR DEVICE**

(30) Priority: 04.12.2023 JP 2023204577
(71) Applicant: FUJI-FILM Corporation, Minato-ku Tokyo 106-8620 (JP)
(72) Inventor: MACHII, Yusuke, Tokyo, 106-8620 (JP)
(74) Representative: Dehns Germany Partnerschaft mbB

(57) **Abstract**

Provided are a processor device (20) and an operation method of a processor device (20) that can perform puncture in consideration of a difficulty level.

A processor device (20) of a laparoscopic system includes a difficulty level calculation unit (40). The difficulty level calculation unit (40) acquires puncture information related to a puncture candidate position and a puncture target, and calculates the puncture difficulty level in a case of performing the puncture from the puncture candidate position toward the puncture target with a puncture needle (32) by using the acquired puncture information. The puncture difficulty level is calculated by using an angle between a puncture direction and a gravity direction, a size of the puncture target, and a distance from the puncture candidate position to the puncture target.

## Description

### BACKGROUND OF THE INVENTION

### 1. Field of the Invention

The present invention relates to a processor device and an operation method of a processor device.

### 2. Description of the Related Art

In the medical field, there is a case where a treatment such as injection of a drug solution or collection of a sample is performed by puncturing a target (puncture target) from a surface of a puncture target part, and a technique for guiding such puncturing is also known. For example, JP2017-169786A describes a configuration of guiding a puncture in a system using an ultrasonic endoscope, and JP2022-080023A describes a configuration of guiding a puncture in a system using a laparoscope.

### SUMMARY OF THE INVENTION

However, in the related art, there is a problem that the puncture cannot be performed in consideration of the difficulty level. That is, it is possible to perform the puncture from the different puncture positions (positions where the needle is inserted) such as performing the puncture from the right side or performing the puncture from the left side even in a case where the puncture targets are the same. As a result, the puncture difficulty level also varies depending on the puncture position. However, in the related art, the difficulty level has not been considered. Therefore, there is a case where a user performs a puncture with a high difficulty level without noticing that a puncture with a lower difficulty level is possible.

The present invention has been made in view of the above background, and an object of the present invention is to provide a processor device and an operation method of a processor device capable of performing puncture in consideration of a difficulty level.

In order to solve the above-described problems, a processor device according to an aspect of an exemplary embodiment of the invention acquires puncture information related to a puncture candidate position on a surface of a puncture target part and a puncture target inside the puncture target part, and calculates a puncture difficulty level from the puncture candidate position to the puncture target by using the puncture information.

A notification of the puncture difficulty level may be performed.

The notification may be performed by displaying the puncture candidate position and the puncture difficulty level in a superimposed manner on an optical image obtained by imaging the puncture target part.

The notification may be performed by displaying the puncture candidate position and the puncture difficulty level in a superimposed manner on a three-dimensional image including the puncture target part.

The puncture difficulty level may be calculated for a plurality of the puncture candidate positions.

The puncture information may be acquired by using an ultrasound image obtained from a reflected wave of an ultrasound wave emitted from the surface of the puncture target part toward the inside.

The puncture difficulty level may be calculated by using an angle between a gravity direction in which gravity occurs and a puncture direction from the puncture candidate position toward the puncture target.

The puncture difficulty level may be calculated by using at least one of a distance from the puncture candidate position to the puncture target or a size of the puncture target.

The puncture difficulty level may be calculated by using information on a blood vessel in the puncture target part.

In addition, in order to solve the above-described problems, an operation method of a processor device according to an exemplary embodiment of the invention comprises a step of acquiring puncture information related to a puncture candidate position on a surface of a puncture target part and a puncture target inside the puncture target part, and a step of calculating a puncture difficulty level from the puncture candidate position to the puncture target by using the puncture information.

According to the exemplary embodiments of the invention, it is possible to perform the puncture in consideration of the difficulty level.

### BRIEF DESCRIPTION OF THE DRAWINGS

Fig. 1 is a schematic view of a laparoscopic system.
Fig. 2 is an explanatory diagram of a puncture guide.
Fig. 3 is a block diagram showing a configuration of a processor device.
Figs. 4A and 4B are explanatory diagrams illustrating a method of calculating a puncture difficulty level.
Figs. 5A and 5B are explanatory diagrams illustrating a method of calculating a puncture difficulty level.
Fig. 6A and 6B are explanatory diagrams illustrating a method of calculating a puncture difficulty level.
Fig. 7 is a flowchart showing a procedure for calculating a puncture difficulty level.
Fig. 8 is an explanatory diagram showing a notification mode of a puncture difficulty level.
Fig. 9 is an explanatory diagram showing a notification mode of a puncture difficulty level.
Fig. 10A and 10B are explanatory diagrams illustrating a method of calculating a puncture difficulty level.
Fig. 11A and 11B are explanatory diagrams illustrating a method of calculating a puncture difficulty level.

### DESCRIPTION OF THE PREFERRED EMBODIMENTS

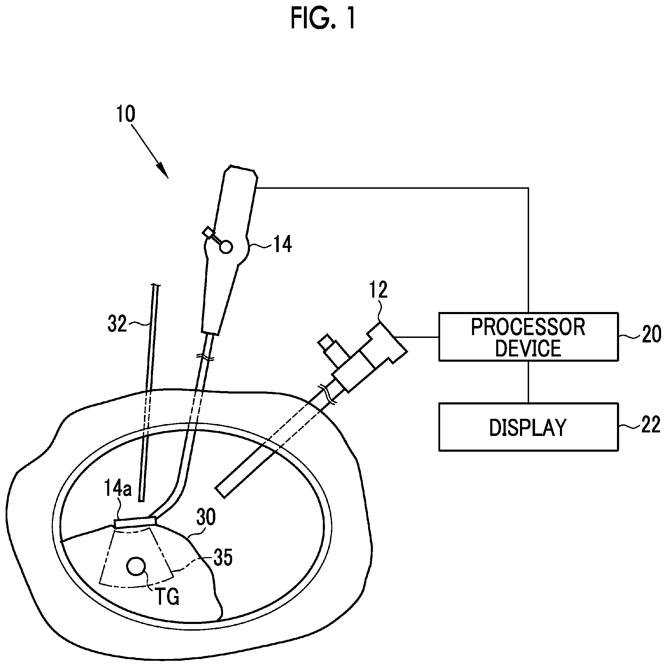

Hereinafter, a case where an exemplary embodiment of the invention is applied to a processor device 20 of a laparoscopic system 10 shown in Fig. 1 will be described. As shown in Fig. 1, the laparoscopic system 10 includes a laparoscope (rigid endoscope) 12 and an ultrasonic endoscope 14, and these are connected to the processor device 20.

The laparoscope 12 is provided with an illumination light irradiation window and a light-receiving window at a distal end portion thereof, receives reflected light of illumination light emitted from the illumination light irradiation window through the light-receiving window, and acquires an optical image generated by using the received reflected light. The acquisition of the optical image is repeatedly performed in a predetermined cycle.

The ultrasonic endoscope 14 is provided with an ultrasound probe 14a at the distal end portion thereof, and acquires an ultrasound image generated by using a reflected wave of an ultrasound wave generated from the ultrasound probe 14a. The acquisition of the ultrasound image is repeatedly performed in a predetermined cycle.

The optical image obtained by the laparoscope 12 and the ultrasound image obtained by the ultrasonic endoscope 14 are input to the processor device 20 and displayed on a display 22. As described above, the acquisition of the optical image and the ultrasound image is repeatedly performed, and a new image is sequentially input to the processor device 20. The processor device 20 updates the display of the display 22 by displaying the newly input image on the display 22 each time a new image is input.

In the laparoscopic system 10, the optical image on the surface of the puncture target part 30 is acquired by the laparoscope 12, and the ultrasound image inside the puncture target part 30 is acquired by the ultrasonic endoscope 14. Then, the operator (doctor) performs a treatment such as puncturing the puncture target TG inside the puncture target part 30 with a puncture needle 32 by using the optical image and the ultrasound image.

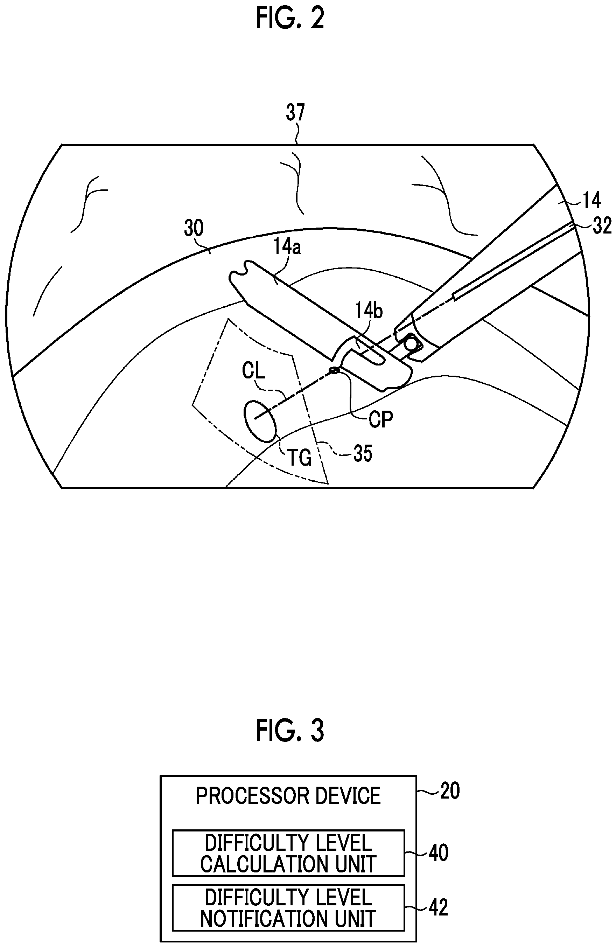

As shown in Fig. 2, the ultrasonic endoscope 14 is provided with a puncture guide 14b formed in a tubular shape, and the puncture is performed around a center line CL of the puncture guide 14b by performing the puncture according to the puncture guide 14b (performing the puncture by inserting the puncture needle 32 into the puncture guide 14b). Then, by performing the puncture around the center line CL of the puncture guide 14b, it is possible to perform the puncture within the imaging range of an ultrasound image 35, that is, to perform the puncture while observing the puncture needle 32 shown in the ultrasound image 35.

In addition, as shown in Fig. 2, in the present embodiment, the ultrasound image 35 is superimposed on an optical image 37. As a result, in the optical image 37, the puncture target part 30 is displayed as if the inside thereof is seen through. Specifically, the processor device 20 analyzes the optical image 37 and detects the imaging range (position, angle, size, or the like) of the ultrasound image 35 from the position or the posture of the ultrasonic endoscope 14 (ultrasound probe 14a) shown in the optical image 37. Then, the position, the angle, and the size of the ultrasound image 35 are adjusted to match the detected imaging range, and the ultrasound image 35 is superimposed on the optical image 37 and the superimposed image is displayed on the display 22. In this manner, it is easy to intuitively understand the relationship (position or posture) between the puncture target part 30, the ultrasonic endoscope 14 (ultrasound probe 14a), and the puncture needle 32, and more appropriate puncture is possible.

In order to more accurately detect the position or the posture of the ultrasonic endoscope 14 (ultrasound probe 14a), a marker such as an AR marker may be provided on the ultrasonic endoscope 14 (ultrasound probe 14a), and the position or the posture of the ultrasonic endoscope 14 (ultrasound probe 14a) may be detected by using the marker shown in the optical image 37. Of course, a configuration may be adopted in which a marker is provided on the puncture needle 32, and the position or the posture of the puncture needle 32 is detected more accurately. In addition, a configuration may be adopted in which a camera is provided in addition to the laparoscope 12 within and/or outside the abdominal cavity, and the camera detects not only the position or the posture of the laparoscope 12 but also the position or the posture of the ultrasonic endoscope 14 (ultrasound probe 14a) or the puncture needle 32. Of course, a configuration may be adopted in which such a camera is provided and a marker is provided on the laparoscope 12 to more accurately detect the position or the posture of the laparoscope 12.

As shown in Fig. 3, the processor device 20 is provided with a difficulty level calculation unit 40 and a difficulty level notification unit 42. The difficulty level calculation unit 40 calculates the puncture difficulty level using the ultrasound image obtained by the ultrasonic endoscope 14. In the present embodiment, the difficulty level calculation unit 40 calculates the difficulty level in a case where the puncture is performed according to the puncture guide 14b at the position and the posture of the ultrasonic endoscope 14 (ultrasound probe 14a) when the ultrasound image used to calculate the difficulty level is acquired.

In the calculation of the difficulty level, the difficulty level calculation unit 40 specifies the puncture position (puncture candidate position) for which the difficulty level is calculated. Specifically, a puncture position (intersection between the center line CL of the puncture guide 14b and the surface of the puncture target part 30) in a case where the puncture is performed according to the puncture guide 14b is specified as a puncture candidate position CP (see Figs. 2 and 4A to 6B) from the position and the posture of the ultrasound probe 14a in the ultrasound image used to calculate the difficulty level. Then, the difficulty level calculation unit 40 calculates the difficulty level in a case of performing the puncture from the puncture candidate position CP toward the puncture target TG with the puncture needle 32. The puncture target TG is specified by the processor device 20 analyzing the ultrasound image or the operator designating the puncture target TG by marking the ultrasound image.

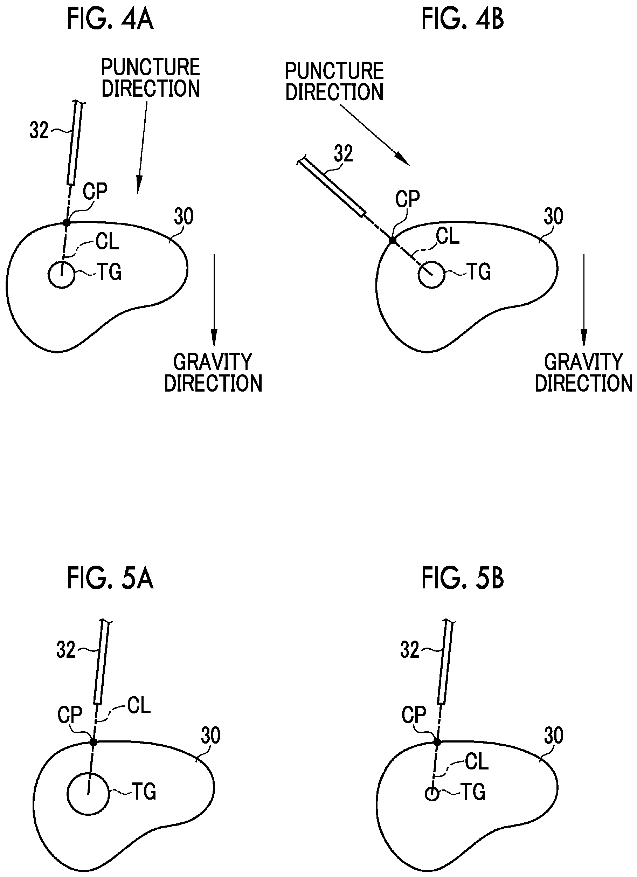

The difficulty level is calculated using a predetermined calculation algorithm. In the present embodiment, the calculation algorithm is generated such that the closer the angle between the puncture direction (direction from the puncture candidate position CP toward the puncture target TG) and the gravity direction (direction in which the gravity occurs) is to 90 degrees, the higher the difficulty level is calculated. Specifically, a calculation algorithm, in which the puncture in the aspect shown in Fig. 4B is calculated to have a higher difficulty level than the puncture in the aspect shown in Fig. 4A, is used. This is because the closer the angle between the puncture direction and the gravity direction is to 90 degrees, the greater the influence of the gravity (the deformation or displacement of the puncture target part 30, the deformation of the puncture needle 32 due to the deformation or displacement of the puncture target part 30, or the like) is, and the puncture difficulty level is increased.

In addition, in the present embodiment, the calculation algorithm is generated such that the smaller the size of the puncture target TG, the higher the difficulty level is calculated. Specifically, a calculation algorithm, in which the puncture in the aspect shown in Fig. 5B is calculated to have a higher difficulty level than the puncture in the aspect shown in Fig. 5A, is used. This is because the smaller the size of the puncture target TG, the narrower the range of deviation (deviation of the puncture position or the puncture angle) allowed for the ideal puncture mode (puncture position (puncture candidate position) or puncture target), and the puncture difficulty level is increased.

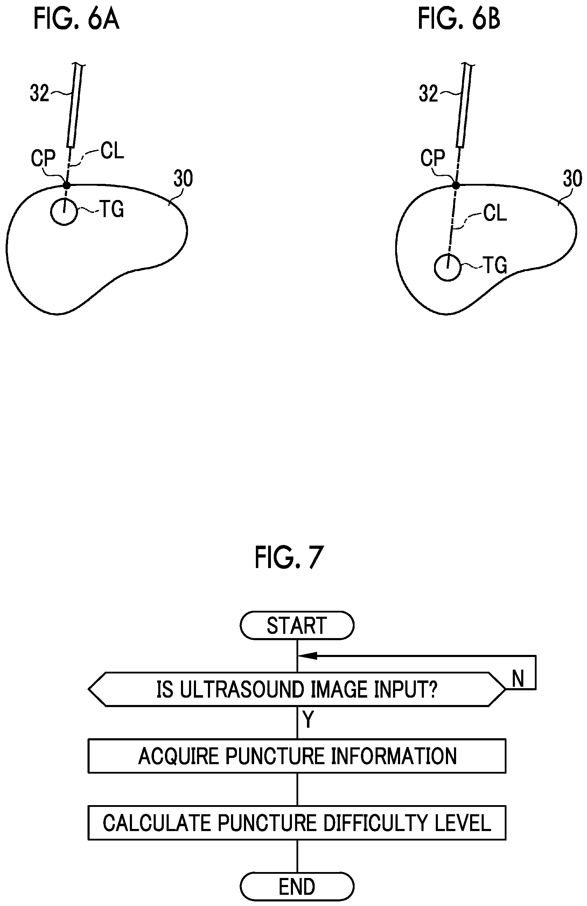

Further, in the present embodiment, the calculation algorithm is generated such that the farther the distance from the puncture candidate position CP to the puncture target TG, the higher the difficulty level is calculated. Specifically, a calculation algorithm, in which the puncture in the aspect shown in Fig. 6B is calculated to have a higher difficulty level than the puncture in the aspect shown in Fig. 6A, is used. This is because, as the distance for performing the puncture is longer, the range of acceptable error from the ideal puncture path is narrowed.

The difficulty level calculation unit 40 detects the angle between the puncture direction and the gravity direction, the size of the puncture target TG, and the distance from the puncture candidate position CP to the puncture target TG by analyzing the ultrasound image, and calculates the difficulty level by substituting these values into the above-described calculation algorithm. As described above, the difficulty level calculation unit 40 performs a step of acquiring the puncture information related to the puncture candidate position CP and the puncture target TG, and a step of calculating the puncture difficulty level by using the puncture information.

As shown in Fig. 7, in a case where the ultrasound image is input from the ultrasonic endoscope 14, the difficulty level calculation unit 40 calculates the above-described difficulty level using the input ultrasound image. As described above, the acquisition of the ultrasound image is repeatedly performed in a predetermined cycle, and new ultrasound images are sequentially input. The difficulty level calculation unit 40 repeatedly performs the calculation of the difficulty level each time a new ultrasound image is input. In this manner, the ultrasonic endoscope 14 (ultrasound probe 14a) scans the surface of the puncture target part 30 to change the position or the posture, so that the puncture difficulty level at the plurality of puncture candidate positions CP is calculated.

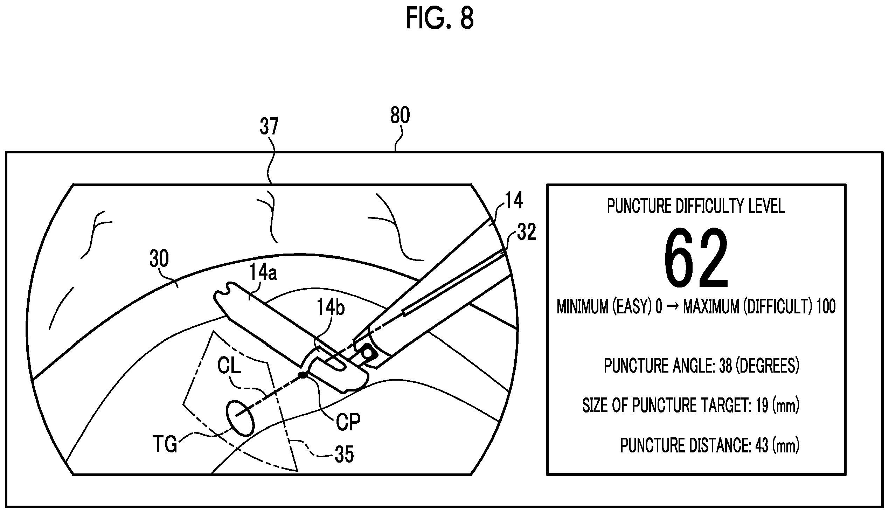

With reference to Fig. 3, the difficulty level notification unit 42 performs the notification of the puncture difficulty level calculated by the difficulty level calculation unit 40. As the notification method, it is considered to perform the notification of the difficulty level by a voice output from a speaker. In addition, for example, as shown in Fig. 8, the difficulty level may be displayed as character information. In Fig. 8, on a display screen 80 of the display 22, the difficulty level (the puncture difficulty level in a case where the puncture is performed with the puncture needle 32 according to the puncture guide 14b at the position and the posture of the ultrasonic endoscope 14 (ultrasound probe 14a) shown in the optical image 37) is displayed side by side with the optical image 37. Further, in Fig. 8, in addition to the puncture difficulty level, information (puncture angle (the angle between the puncture direction and the gravity direction), puncture target size (the size of the puncture target TG), and puncture distance (the distance from the puncture candidate position CP to the puncture target TG)) that is the basis of the calculation of the puncture difficulty level is also displayed.

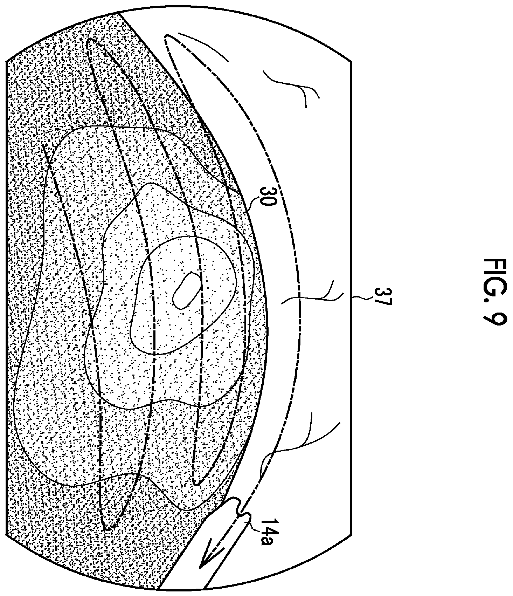

As described above, the puncture difficulty level at the plurality of puncture candidate positions is calculated by allowing the ultrasonic endoscope 14 (ultrasound probe 14a) to scan the surface of the puncture target part 30 to change the position or the posture. Therefore, as shown in Fig. 9, the notification may be performed by displaying the puncture difficulty level of each of the plurality of puncture candidate positions in a superimposed manner on the optical image 37. In Fig. 9, in a case where the ultrasonic endoscope 14 (ultrasound probe 14a) scans a path indicated by a two-point chain line, the puncture difficulty level at each of the plurality of puncture candidate positions is calculated in the process of the scanning, and the optical image 37 is colored such that the color becomes thicker at the point (puncture candidate position) in which the puncture difficulty level is higher. As a result, the notification of the puncture difficulty level at each puncture candidate position is performed.

In addition, in the above-described embodiment, the configuration has been described in which the puncture difficulty level is calculated by using the ultrasound image obtained by the ultrasonic endoscope 14, as an example, but the embodiment of the present invention is not limited to thereto. It is also possible to acquire the three-dimensional image of the puncture target part 30 or the puncture target TG in advance (before a treatment such as surgery using the laparoscopic system 10) by computed tomography (CT), magnetic resonance imaging (MRI), or the like, and the puncture difficulty level may be calculated by using the three-dimensional image obtained in advance in this way. In a case where the puncture difficulty level is calculated by using the three-dimensional image, the three-dimensional image is colored such that the color becomes thicker at the point (puncture candidate position) in which the puncture difficulty level is higher. As a result, the notification of the puncture difficulty level at each puncture candidate position can be performed.

In the above-described embodiment, the example, in which the puncture difficulty level is calculated, has been described by using three pieces of information, which are the puncture angle (the angle between the puncture direction and the gravity direction), the puncture target size (the size of the puncture target TG), and the puncture distance (the distance from the puncture candidate position CP to the puncture target TG), but the embodiment of the present invention is not limited thereto. The puncture difficulty level may be calculated by using any one of the three or a combination of two of the three, without using all of the three. In addition to these three elements or instead of some or all of these three elements, the puncture difficulty level may be calculated by using elements other than these three elements.

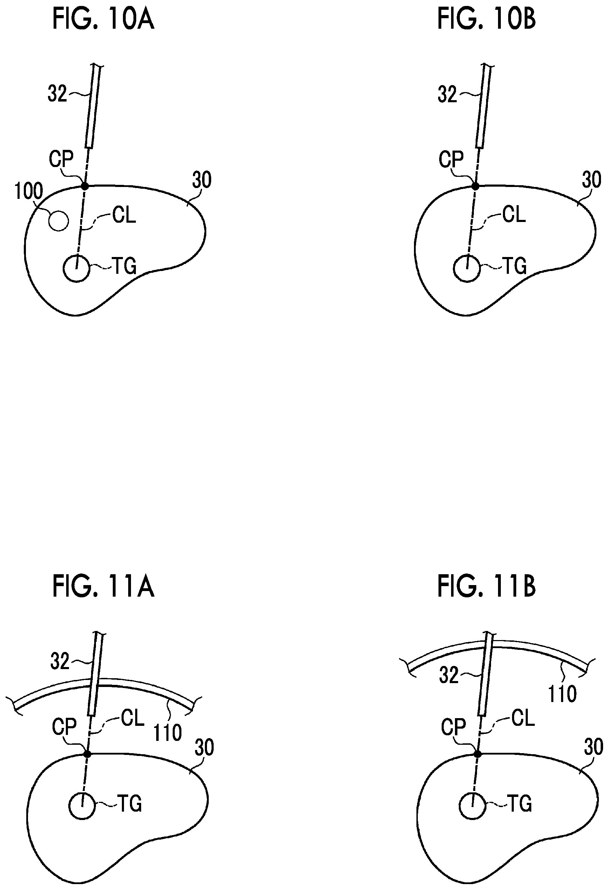

In a case where the difficulty level is calculated by using elements other than the three elements described above, for example, with the information of the puncture avoidance part (a part where a puncture in a blood vessel or the like is avoided), a calculation algorithm is created in which the puncture difficulty level is increased as the puncture avoidance part is closer to the puncture path (a route connecting the puncture candidate position CP and the puncture target TG) and/or the number of puncture avoidance parts within a predetermined distance from the puncture path is increased. Specifically, a calculation algorithm is created such that a case where there is a puncture avoidance part (in the present example, the blood vessel 100) in the vicinity of the puncture path as in the aspect shown in Fig. 10B is calculated to have a higher difficulty level than a case where there is no puncture avoidance part in the vicinity of the puncture path as in the aspect shown in Fig. 10A. Then, the puncture difficulty level may be calculated by using this calculation algorithm.

In addition, in a case where the difficulty level is calculated by using elements other than the three elements described above, for example, it is considered to use a distance between the peritoneum 110 and the puncture candidate position CP. Specifically, in a case where the distance between the peritoneum 110 and the puncture candidate position CP is relatively long as in the aspect shown in Fig. 11B, the puncture needle 32 is more likely to be affected by gravity (the puncture needle 32 is more likely to be deformed (deflected)) than in a case where the distance between the peritoneum 110 and the puncture candidate position CP is relatively short as in the aspect shown in Fig. 11A. Therefore, a calculation algorithm is created such that the difficulty level of the case in Fig. 11B is calculated to be higher. Then, the puncture difficulty level may be calculated by using this calculation algorithm.

The distance between the peritoneum 110 and the puncture candidate position CP can be acquired, for example, by analyzing the optical image 37. In addition, the distance between the peritoneum 110 and the puncture candidate position CP also can be calculated with the pneumoperitoneum simulation in which the position or the posture of the ultrasonic endoscope 14 (ultrasound probe 14a) and the puncture needle 32 detected from the optical image 37, and the three-dimensional image obtained in advance are used. Further, the distance between the peritoneum 110 and the puncture candidate position CP can also be calculated by a stereo matching technique using two images (parallax images) in which the common target (peritoneum 110 and puncture candidate position CP) is imaged at different angles. In this case, the parallax image can also be acquired by performing imaging a plurality of times with a single-lens laparoscope while changing a position (angle) in addition to being obtained by a multiple-lens laparoscope.

In the above-described embodiment, the example in which the exemplary embodiment of the invention is applied to the processor device 20 of the laparoscopic system 10 has been described, and the exemplary embodiment of the invention may be applied to a processor device of a system other than the laparoscopic system 10. In addition, for example, a processor device for image processing may be provided in addition to the laparoscopic system 10, and the processor device for image processing may function as the processor device of the exemplary embodiment of the invention. In this case, a configuration may be adopted in which the optical image obtained by the laparoscope 12 and the ultrasound image obtained by the ultrasonic endoscope 14 are input to the processor device for image processing, and the processor device for image processing functions as the difficulty level calculation unit 40 or the difficulty level notification unit 42 described above to calculate the difficulty level and perform the notification.

In the above-described embodiment, a hardware structure of a processing unit that executes various types of processing, such as the difficulty level calculation unit 40 and the difficulty level notification unit 42, is various processors as shown below. The various processors include a central processing unit (CPU) which is a general-purpose processor functioning as various processing units by executing software (program), a programmable logic device (PLD) such as a field programmable gate array (FPGA) which is a processor having a circuit configuration changeable after manufacture, a dedicated electric circuit which is a processor having a circuit configuration dedicatedly designed to execute various types of processing, and the like.

One processing unit may be configured by one of these various processors, or may be configured by a combination of two or more processors of the same or different kinds (for example, a combination of a plurality of FPGAs or a combination of a CPU and an FPGA). Further, one processor may constitute a plurality of processing units. As an example in which one processor constitutes a plurality of processing units, first, there is a form in which one processor is configured by a combination of one or more CPUs and software and the processor functions as the plurality of processing units, as represented by a computer such as a client and a server. Second, there is a form in which a processor, which implements functions of the entire system including the plurality of processing units with one integrated circuit (IC) chip, is used, as represented by a system on chip (SoC), or the like. As described above, various processing units are configured using one or more of the above-described various processors as hardware structures.

Further, the hardware structures of these various processors are, more specifically, electric circuits (circuitry) in a form in which circuit elements such as semiconductor elements are combined. In addition, a hardware structure of the storage unit is a storage device such as a hard disc drive (HDD) and a solid state drive (SSD).

### Explanation of References

10: laparoscopic system
12: laparoscope
14: ultrasonic endoscope
14a: ultrasound probe
14b: puncture guide
20: processor device
22: display
30: puncture target part
32: puncture needle
35: ultrasound image
37: optical image
40: difficulty level calculation unit
42: difficulty level notification unit
80: display screen
100: blood vessel
110: peritoneum
TG: puncture target
CL: center line
CP: puncture candidate position

## Claims

1. A processor device (20) configured to:
acquire puncture information related to a puncture candidate position on a surface of a puncture target part (30) and a puncture target inside the puncture target part (30); and
calculate a puncture difficulty level of puncturing from the puncture candidate position to the puncture target by using the puncture information.

2. The processor device (20) according to claim 1,
wherein the puncture difficulty level is notified.

3. The processor device (20) according to claim 2,
wherein the notification is performed by displaying the puncture candidate position and the puncture difficulty level superimposed on an optical image (37) obtained by imaging the puncture target part (30).

4. The processor device (20) according to claim 2,
wherein the notification is performed by displaying the puncture candidate position and the puncture difficulty level superimposed on a three-dimensional image that includes the puncture target part (30).

5. The processor device (20) according to any one of claims 1 to 4,
wherein the puncture difficulty level is calculated for a plurality of the puncture candidate positions.

6. The processor device (20) according to any one of claims 1 to 4,
wherein the puncture information is acquired by using an ultrasound image obtained from a reflected wave of an ultrasound wave emitted from the surface of the puncture target part toward its interior.

7. The processor device (20) according to any one of claims 1 to 4,
wherein the puncture difficulty level is calculated by using an angle between a gravity direction in which gravity occurs and a puncture direction from the puncture candidate position toward the puncture target.

8. The processor device (20) according to claim 7,
wherein the puncture difficulty level is calculated by using at least one of a distance from the puncture candidate position to the puncture target and a size of the puncture target.

9. The processor device (20) according to claim 8,
wherein the puncture difficulty level is calculated by using information on blood vessels in the puncture target part (30).

10. An operation method of a processor device (20), the method comprising:
acquiring puncture information related to a puncture candidate position on a surface of a puncture target part (30) and a puncture target inside the puncture target part (30); and
calculating a puncture difficulty level of puncturing from the puncture candidate position to the puncture target by using the puncture information.
